# EUROPEAN PATENT SPECIFICATION

(11) **EP 3 437 630 B1**
(45) Date of publication and mention of the grant of the patent: **11.11.2020**
(21) Application number: 17774737.5
(22) Date of filing: 24.03.2017
(51) Int. Cl.: A61K 8/81, A61K 8/42, A61Q 5/02, A61Q 19/10, C08F 220/06

(54) **GEL COMPOSITION, COSMETIC AND MANUFACTURING METHOD FOR GEL COMPOSITION**
GELZUSAMMENSETZUNG UND HERSTELLUNGSVERFAHREN FÜR DIE GELZUSAMMENSETZUNG
COMPOSITION DE GEL, PRODUIT COSMÉTIQUE ET PROCÉDÉ DE FABRICATION D'UNE COMPOSITION DE GEL

(30) Priority: 31.03.2016 JP 2016072397; 09.06.2016 JP 2016115203
(43) Date of publication of application: 06.02.2019
(73) Proprietor: Sumitomo Seika Chemicals Co., Ltd., Kako-gun, Hyogo 675-0145 (JP)
(72) Inventor: IZAWA, Shingo, Himeji-shi Hyogo 672-8076 (JP); MURAKAMI, Ryosuke, Himeji-shi Hyogo 672-8076 (JP); NISHIGUCHI, Satoshi, Himeji-shi Hyogo 672-8076 (JP); IKEDA, Miyu, Himeji-shi Hyogo 672-8076 (JP); NAKASHIMA, Rie, Himeji-shi Hyogo 672-8076 (JP)
(74) Representative: Mewburn Ellis LLP
(86) International application number: PCT/JP2017/011953
(87) International publication number: WO 2017/170200

(56) References cited:
- WO-A1-2007/055354
- WO-A1-2014/007370
- WO-A1-2014/021434
- JP-A- 2009 084 502
- JP-A- 2009 084 502
- JP-A- 2010 235 833
- JP-A- 2011 213 676
- US-A1- 2016 045 424

## Description

### Technical Field

The present invention relates to a gel composition that can be suitably used as a thickener for cosmetic preparations in the form of viscous solutions or gels used for, for example, hair care products such as shampoos or skin care products such as body washes and facial cleansers, a method for producing the gel composition, and a cosmetic preparation comprising the gel composition.

### Background Art

As thickeners for cosmetic preparations, for example, natural materials such as xanthan gum, semi-synthetic materials such as hydroxyethyl cellulose, and synthetic materials such as carboxyvinyl polymers and alkyl-modified carboxyvinyl polymers have been extensively used. In particular, carboxyl group-containing polymers such as carboxyvinyl polymers and alkyl-modified carboxyvinyl polymers are used for various cosmetic preparations, because they exhibit excellent thickening properties even when used in small amounts, and can control the after-feel of the cosmetic preparation.

Those known as such carboxyl group-containing polymers include alkyl-modified carboxyvinyl polymers such as, for example, a carboxyvinyl polymer obtained by reacting acrylic acid and a pentaerythritol allyl ether as a crosslinking agent in a specific solvent mixture (see Patent Literature 1), a copolymer obtained by reacting a specific amount of an olefinic unsaturated carboxylic acid monomer and a specific amount of a (meth)acrylic acid alkyl ester (in which the alkyl group has 10 to 30 carbon atoms) (see Patent Literature 2), a copolymer obtained by reacting a specific amount of an olefinic unsaturated carboxylic acid monomer, a specific amount of a (meth)acrylic acid alkyl ester (in which the alkyl group has 10 to 30 carbon atoms), and a crosslinking agent (see Patent Literature 3), and a copolymer obtained by reacting an olefinic unsaturated carboxylic acid monomer and a (meth)acrylic acid alkyl ester (in which the alkyl group has 8 to 30 carbon atoms) (see Patent Literature 4). Typically, these carboxyl group-containing polymers are dissolved in water or the like and then neutralized with an alkaline component to give about 0.1 to 1% by mass neutral viscous solutions, which are used for cosmetic preparations.

These neutral viscous solutions are disadvantageous in that the viscosity or stability decreases, or a portion of the polymer precipitates, under the influence of an emulsifier that is a main ingredient of cosmetic preparations. In particular, in the field of cosmetic preparations, cosmetic preparations that take into account the safety and mildness to the skin, in addition to various characteristics such as the after-feel, have recently attracted attention. In particular, emulsifiers with naturally occurring coconut oil fatty acid-based betaine structures have been of interest as cleansing ingredients, and gel compositions containing high concentrations of these ingredients are desired.

Among these emulsifiers with coconut oil fatty acid-based betaine structures, cocamidopropyl betaine has excellent detergency, while taking into account the safety and mildness to the skin. It is mildly irritating and safe enough to be used as baby shampoos, and is used in cosmetic preparations for cleansing, such as shampoos. Patent literature 5 and 6 disclose cosmetic compositions containing alkyl-modified carboxyvinyl polymers and betaines.

### Citation List

### Patent Literature

Patent Literature 1: US Patent No. 5,342,911
Patent Literature 2: JP 51-6190 A
Patent Literature 3: JP 59-232107 A
Patent Literature 4: Specification of US Patent No. 5,004,598 Patent literature 5: JP 2009-84502
Patent literature 6: US 2016/0045424

### Summary of Invention

### Technical Problem

It is a main object of the present invention to provide a gel composition that comprises a betaine-based emulsifier and can be suitably used as a thickener for cosmetic preparations and the like, a method for producing the gel composition, and a cosmetic preparation comprising the gel composition.

### Solution to Problem

The inventors of the present invention conducted extensive research to solve the aforementioned problem. As a result, they found that a gel composition comprising 0.1 to 2% by mass of a neutralized product of an alkyl-modified carboxyl group-containing polymer, which is a copolymer of monomers comprising 100 parts by mass of (meth)acrylic acid (a), 2.5 to 5 parts by mass of a (meth)acrylic acid alkyl ester (b) in which the alkyl group has 18 to 24 carbon atoms, and 0.1 part by mass or less of a compound (c) having two or more ethylenically unsaturated groups, and a betaine-based emulsifier, wherein the content of the betaine-based emulsifier is 0.2 to 0.8% by mass and the pH of the composition is 4.4 to 5.5, exhibits excellent thickening properties, and can be suitably used as a thickener for cosmetic preparations and the like.

In summary, the present invention provides aspects of the invention comprising the following features:
Item 1. A gel composition comprising:
   0.1 to 2% by mass of a neutralized product of an alkyl-modified carboxyl group-containing polymer, which is a copolymer of monomers comprising 100 parts by mass of (meth)acrylic acid (a), 2.5 to 5 parts by mass of a (meth)acrylic acid alkyl ester (b) in which the alkyl group has 18 to 24 carbon atoms, and 0.1 part by mass or less of a compound (c) having two or more ethylenically unsaturated groups; and
   a betaine-based emulsifier in an amount of 0.2 to 0.8% by mass, the pH of the composition being 4.4. to 5.5.
Item 2. The gel composition according to item 1, which has a viscosity at 25°C of 50,000 mPa·s or more when the composition has the following composition:
   (composition)
   a composition comprising 96 parts by mass of a 1% by mass neutral viscous solution of the alkyl-modified carboxyl group-containing polymer; and 4 parts by mass of an aqueous solution of the betaine-based emulsifier (having a solids content of about 30% by mass).
Item 3. The gel composition according to item 1 or 2, wherein the alkyl-modified carboxyl group-containing polymer has a viscosity at 25°C of 1500 mPa·s or less and a light transmittance of 90% or more, when in the form of a 1% by mass neutral viscous solution, and
   a solution prepared by adding 0.25 to 3 parts by mass of sodium chloride to 100 parts by mass of the 1% by mass neutral viscous solution has a maximum viscosity at 25°C of 15,000 to 40,000 mPa·s and a light transmittance of 90% or more.
Item 4. The gel composition according to item 1 or 3, wherein the content of the betaine-based emulsifier is 0.2 to 0.8% by mass, and
   the pH of the composition is 4.4 to 5.5.
Item 5. The gel composition according to any one of items 1 to 4, wherein the compound (c) having two or more ethylenically unsaturated groups is at least one selected from the group consisting of pentaerythritol allyl ethers, diethylene glycol diallyl ether, polyethylene glycol diallyl ether, and polyallylsaccharose.
Item 6. The gel composition according to any one of items 1 to 5, wherein the betaine-based emulsifier is cocamidopropyl betaine.
Item 7. A cosmetic preparation comprising the gel composition according to any one of items 1 to 6.
Item 8. A method for producing a gel composition comprising the steps of:
   mixing an alkyl-modified carboxyl group-containing polymer, which is a copolymer of monomers comprising 100 parts by mass of (meth)acrylic acid (a), 2.5 to 5 parts by mass of a (meth)acrylic acid alkyl ester (b) in which the alkyl group has 18 to 24 carbon atoms, and 0.1 part by mass or less of a compound (c) having two or more ethylenically unsaturated groups, with an alkaline component, to prepare a neutralized product of the alkyl-modified carboxyl group-containing polymer; and
   mixing the neutralized product of the alkyl-modified carboxyl group-containing polymer with a betaine-based emulsifier.

### Advantageous Effects of Invention

The present invention can provide a gel composition that comprises a betaine-based emulsifier and can be suitably used as a thickener for cosmetic preparations and the like, and a method for producing the gel composition.

### Description of Embodiments

A gel composition of the present invention comprises a neutralized product of an alkyl-modified carboxyl group-containing polymer and a betaine-based emulsifier, wherein the alkyl-modified carboxyl group-containing polymer is a copolymer of monomers comprising 100 parts by mass of (meth)acrylic acid (a), 2.5 to 5 parts by mass of a (meth)acrylic acid alkyl ester (b) in which the alkyl group has 18 to 24 carbon atoms, and 0.1 part by mass or less of a compound (c) having two or more ethylenically unsaturated groups. The gel composition of the present invention, a method for producing the gel composition, and a cosmetic preparation comprising the gel composition will be hereinafter described in detail.

As used herein, the term "(meth)acrylic acid" collectively refers to "acrylic acid and methacrylic acid". As used herein, the term "neutral viscous solution" refers to a viscous liquid obtained by mixing an aqueous dispersion of an alkyl-modified carboxyl group-containing polymer with an alkaline component (for example, an alkali metal hydroxide such as sodium hydroxide, or an amine such as triethanolamine or diisopropanolamine) such that the pH of the aqueous dispersion of the alkyl-modified carboxyl group-containing polymer is adjusted to a predetermined value (typically a pH of about 3.5 to 6.5). The term "1% by mass neutral viscous solution" refers to a neutral viscous solution containing 1% by mass of a neutralized product of the alkyl-modified carboxyl group-containing polymer.

In the present invention, the neutralized product of the alkyl-modified carboxyl group-containing polymer is a compound obtained by partially or completely neutralizing the alkyl-modified carboxyl group-containing polymer with the above-mentioned alkaline component. In the gel composition of the present invention (in particular, with a pH of about 3.5 to 6.5), the neutralized product of the alkyl-modified carboxyl group-containing polymer is typically a partially neutralized product of the alkyl-modified carboxyl group-containing polymer. Before neutralization, the alkyl-modified carboxyl group-containing polymer typically has a pH of about 2.5 to 3.

The alkyl-modified carboxyl group-containing polymer is a copolymer of 100 parts by mass of (meth)acrylic acid (hereinafter sometimes referred to as the component (a)), 2.5 to 5 parts by mass of a (meth)acrylic acid alkyl ester in which the alkyl group has 18 to 24 carbon atoms (hereinafter sometimes referred to as the component (b)), and 0.1 part by mass or less of a compound having two or more ethylenically unsaturated groups (hereinafter sometimes referred to as the component (c)). Specifically, the alkyl-modified carboxyl group-containing polymer of the present invention is a copolymer of at least the components (a) and (b), as well as optionally the component (c).

In the present invention, at least one of acrylic acid and methacrylic acid can be used as (meth)acrylic acid (the component (a)).

The (meth)acrylic acid alkyl ester in which the alkyl group has 18 to 24 carbon atoms (the component (b)) is an ester of (meth)acrylic acid and a higher alcohol in which the alkyl group has 18 to 24 carbon atoms. Specific examples of the component (b) include an ester of (meth)acrylic acid and stearyl alcohol (i.e., stearyl (meth)acrylate), an ester of (meth)acrylic acid and eicosanol (i.e., eicosanyl (meth)acrylate), an ester of (meth)acrylic acid and behenyl alcohol (i.e., behenyl (meth)acrylate), and an ester of (meth)acrylic acid and tetracosanol (i.e., tetracosanyl (meth)acrylate). Among the above, stearyl methacrylate, eicosanyl methacrylate, behenyl methacrylate, and tetracosanyl methacrylate, for example, can be suitably used, because they can achieve an excellent thickening effect obtained by a gel composition having high transparency, even in the presence of the neutralized product of the alkyl-modified carboxyl group-containing polymer and a cationic polymer that is often blended into a cosmetic preparation or the like.

In the alkyl-modified carboxyl group-containing polymer, the monomer-constituting components (b) may be used alone or in combinations of two or more. When two or more components (b) are used for copolymerization, trade name BLEMMER VMA70 from NOF Corporation (a mixture of 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate), for example, can be used.

The proportion of the component (b) in the alkyl-modified carboxyl group-containing polymer is 2.5 to 5 parts by mass per 100 parts by mass of (meth)acrylic acid (the component (a)). In order to effectively increase the viscosity of the gel composition in the presence of the betaine-based emulsifier, the proportion of the component (b) is, for example, preferably 3 to 4 parts by mass per 100 parts by mass of the component (a). If the proportion of the component (b) is less than 2.5 parts by mass per 100 parts by mass of the component (a), the viscosity of the gel composition tends to decrease in the presence of the betaine-based emulsifier, whereas if the proportion is more than 5 parts by mass, the viscosity of the gel composition also tends to decrease.

The compound having two or more ethylenically unsaturated groups (the component (c)) is a compound having two or more polymerizable ethylenically unsaturated groups. In the present invention, the component (c) optionally constitutes a monomer of the alkyl-modified carboxyl group-containing polymer. Preferred specific examples of the component (c) include polyallyl ethers of pentaerythritol such as pentaerythritol diallyl ether, pentaerythritol triallyl ether, and pentaerythritol tetraallyl ether (each of them and mixtures of two or more of them are collectively referred to as "pentaerythritol allyl ethers"), diethylene glycol diallyl ether, polyethylene glycol diallyl ether, and polyallylsaccharose. In the alkyl-modified carboxyl group-containing polymer, the optional monomer-constituting components (c) may be used alone or in combinations of two or more.

The proportion of the component (c) in the alkyl-modified carboxyl group-containing polymer is, for example, 0.1 part by mass or less, preferably 0.0001 to 0.05 part by mass, and more preferably 0.001 to 0.044 part by mass, per 100 parts by mass of (meth)acrylic acid (the component (a)). If the proportion of the component (c) is more than 0.1 part by mass, the viscosity of the gel composition tends to decrease, and the stability of the cosmetic preparation obtained using the gel composition may deteriorate.

The alkyl-modified carboxyl group-containing polymer of the present invention may be copolymerized with a monomer other than the components (a) to (c) (another monomer copolymerizable with at least one of the components (a) to (c)). For example, lauryl (meth)acrylate may be used in a proportion of more than 0 part by mass and 1 part by mass or less, per total 100 parts by mass of the components (a) to (c). In the present invention, although the total proportion of the components (a) to (c) in the monomers constituting the alkyl-modified carboxyl group-containing polymer is not particularly limited, it is, for example, preferably 50% by mass or more, more preferably about 80 to 100% by mass, still more preferably about 90 to 100% by mass, and particularly preferably about 95 to 100% by mass. The total proportion may be substantially 100% by mass.

The method for producing the alkyl-modified carboxyl group-containing polymer is not particularly limited, and examples include a method in which raw materials including at least the components (a) and (b), as well as a polymerization initiator, are added to a solvent to form a solution, and the solution is heated with stirring in an inert gas atmosphere to carry out the polymerization.

Examples of inert gases for creating the inert gas atmosphere include, but are not particularly limited to, nitrogen gas and argon gas.

The solvent is not particularly limited as long as it dissolves at least the components (a) and (b), but does not dissolve the alkyl-modified carboxyl group-containing polymer produced, and does not inhibit the copolymerization reaction. Specific examples of the solvent include hydrocarbon solvents such as normal pentane, normal hexane, normal heptane, cyclopentane, and cyclohexane; and ester-based solvents such as methyl acetate, ethyl acetate, propyl acetate, and butyl acetate. These solvents may be used alone or in combinations of two or more. Among these solvents, normal hexane, normal heptane, and ethyl acetate, for example, are suitably used.

The amount of the solvent to be used is preferably 300 to 5,000 parts by mass per 100 parts by mass of the component (a), in order to improve the ease of stirring, and from an economic viewpoint.

The polymerization initiator is preferably a radical polymerization initiator, for example. Specific examples of the polymerization initiator include α,α'-azoisobutyronitrile, 2,2'-azobis(2,4-dimethylvaleronitrile), and 2,2'-azobis(methylisobutyrate). Among the above, 2,2'-azobis(methylisobutyrate) is preferred in order to obtain an alkyl-modified carboxyl group-containing polymer having a high molecular weight. These polymerization initiators may be used alone or in combinations of two or more.

The amount of the polymerization initiator to be used is preferably about 0.00003 to 0.002 mol per mole of the component (a), in order to keep an appropriate reaction rate.

The polymerization temperature is preferably about 50 to 90°C, and more preferably about 55 to 75°C, in order to keep an appropriate reaction rate.

The polymerization time is typically about 0.5 to 5 hours, although it may vary depending on the polymerization temperature and the like.

The alkyl-modified carboxyl group-containing polymer can be obtained by removing the solvent from the dispersion after the polymerization, by heating to 80 to 130°C, for example, in order to reduce the drying time to suppress agglomeration of the copolymer. When the heating temperature at this time is 80°C or more, the drying time can be reduced. Moreover, when the heating temperature is 130°C or less, a decrease in the dispersibility of the alkyl-modified carboxyl group-containing polymer in water can be suitably suppressed.

In order to increase the viscosity and transparency of the gel composition even in the presence of the betaine-based emulsifier, the alkyl-modified carboxyl group-containing polymer (in the absence of the betaine-based emulsifier) preferably has a viscosity at 25°C of 1500 mPa·s or less and a light transmittance of 90% or more, when in the form of a 1% by mass neutral viscous solution. More preferably, the viscosity is 100 to 1000 mPa·s, and the light transmittance is 96% or more. Furthermore, a solution prepared by adding 0.25 to 3 parts by mass of sodium chloride to 100 parts by mass of the 1% by mass neutral viscous solution preferably has a maximum viscosity at 25°C of 15,000 to 40,000 mPa·s and a light transmittance of 90% or more. More preferably, the maximum viscosity is 15000 to 26000 mPa·s, and the light transmittance is 96% or more.

In the present invention, each of the terms "viscosity" and "light transmittance" refers to a value measured using the method described in the Examples.

In the gel composition of the present invention, the content of the neutralized product of the alkyl-modified carboxyl group-containing polymer is about 0.1 to 2% by mass, and more preferably about 0.5 to 1.5% by mass.

Preferred examples of the betaine-based emulsifier include the following, in order to increase the viscosity of the gel composition in the presence of the above-described neutralized product of the alkyl-modified carboxyl group-containing polymer: 2-lauryl-N-carboxymethyl-N'-hydroxyethyl imidazolinium betaine; lauramidopropyl hydroxy sulfo betaine; cocamidopropyl betaine; lauramidopropyl betaine; myristamidopropyl betaine; and palm kernel fatty acid amidopropyl betaines. Among the above, cocamidopropyl betaine is preferably used, because it can achieve high foaming when the gel composition of the present invention is blended into a cosmetic preparation, is mildly irritating, and can achieve a conditioning effect. These betaine-based emulsifiers may be used alone or in combinations of two or more.

In the gel composition of the present invention, when the pH of the gel composition of the present invention is set to a lower value (for example, a pH in the range of about 4.4 to 5.5), the content of the aqueous solution of the betaine-based emulsifier is about 0.67 to 2.67% by mass (the content of the betaine-based emulsifier is about 0.2 to 0.8% by mass).

In the gel composition of the present invention, for example, when the amount of the aqueous solution of the betaine-based emulsifier is set to about 0.67 to 2.67% by mass (the content of the betaine-based emulsifier is about 0.2 to 0.8% by mass), a gel composition having a high viscosity even at a low pH in the range of 4.4 to 5.5 can be obtained, and the gel composition can be suitably used as a thickener for cosmetic preparations and the like. Furthermore, in the gel composition of the present invention, the difference in viscosity before and after addition of the betaine-based emulsifier is particularly large in this low pH range. Thus, a gel composition having a suitably high viscosity in the low pH range can be obtained by mixing various components homogeneously at a low viscosity before addition of the betaine-based emulsifier, and then adding the betaine-based emulsifier.

The gel composition of the present invention preferably has a viscosity at 25°C of 50,000 mPa·s or more, and more preferably 100,000 mPa·s or more, when the composition has the composition shown below. Although the upper limit of the viscosity is not particularly limited since it varies depending on the use or purpose, it is preferably 200,000 mPa·s or less, and more preferably 150,000 mPa·s or less.

### (Composition)

A composition comprising 96 parts by mass of a 1% by mass neutral viscous solution of the alkyl-modified carboxyl group-containing polymer; and 4 parts by mass of the aqueous solution of the betaine-based emulsifier (having a solids content of about 30% by mass, for example, 28 to 33% by mass, and particularly 31.5% by mass).

Furthermore, in the gel composition of the present invention, when the pH is low, i.e., from 4.4 to 5.5, a viscosity of, for example, 20,000 mPa·s or more, and even 53,000 mPa·s or more can be achieved by setting the content of the betaine-based emulsifier to 0.2 to 0.8% by mass. In this case, the upper limit of the viscosity is typically about 200,000 mPa·s.

Although a detailed mechanism by which the gel composition of the present invention has a high viscosity and can be suitably used as a thickener for cosmetic preparations and the like is not necessarily clear, it can be assumed to be as follows, for example: In the present invention, because the neutralized product of the alkyl-modified carboxyl group-containing polymer contained in the gel composition is obtained by copolymerizing the components (a) to (c) in the above-described predetermined amounts, the neutralized product of the alkyl-modified carboxyl group-containing polymer and the betaine-based emulsifier suitably form aggregates in water with an increased thickness, thus forming a gel composition having a high viscosity.

The gel composition of the present invention typically contains water in addition to the components (a) to (c). Although the content of water in the gel composition of the present invention is not particularly limited, it is preferably 91.4 to 99.3% by mass, and more preferably 92.0 to 99.0% by mass.

The gel composition of the present invention may also contain various additives, such as other thickeners, alcohols, pH adjusters, moisturizers, oils, salts, anionic surfactants, nonionic surfactants, cationic surfactants, chelating agents, preservatives, antioxidants, ultraviolet absorbers, colorants, and perfumes, as long as they do not impair the purpose of the composition. The gel composition of the present invention can be suitably used as a thickener for cosmetic preparations in the form of viscous solutions or gels used for, for example, hair care products such as shampoos or skin care products such as body washes and facial cleansers. The cosmetic preparation of the present invention contains the gel composition of the present invention, and may further contain various cosmetic ingredients to be blended into cosmetic preparations.

Although the method for producing the gel composition of the present invention is not particularly limited, the gel composition can be suitably produced using, for example, a method including the following steps:
mixing the alkyl-modified carboxyl group-containing polymer, which is a copolymer of monomers comprising 100 parts by mass of (meth)acrylic acid (a), 2.5 to 5 parts by mass of a (meth)acrylic acid alkyl ester (b) in which the alkyl group has 18 to 24 carbon atoms, and 0.1 part by mass or less of a compound (c) having two or more ethylenically unsaturated groups, with the alkaline component, to prepare a neutralized product of the alkyl-modified carboxyl group-containing polymer; and
mixing the neutralized product of the alkyl-modified carboxyl group-containing polymer with the betaine-based emulsifier.

In the above-described method, the pH of the gel composition can be adjusted to a desired value by adding the alkaline component at least either before or after mixing of the betaine-based emulsifier.

The method for producing the gel composition of the present invention may further include mixing various additives. Mixing of the various additives can be performed either before or after mixing of the neutralized product of the alkyl-modified carboxyl group-containing polymer and the betaine-based emulsifier.

### Examples

The present invention will be hereinafter described in detail by way of examples and comparative examples, although the present invention is not limited to the examples.

### (Production Example 1)

A 500-mL four-necked flask equipped with a stirrer, a thermometer, a nitrogen inlet tube, and a condenser tube was charged with 45 g (0.625 mol) of acrylic acid, 1.4 g of BLEMMER VMA70 (NOF Corporation; a mixture containing 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate), 0.02 g of a pentaerythritol allyl ether (a mixture of pentaerythritol triallyl ether and pentaerythritol tetraallyl ether), 150 g of normal hexane, and 0.081 g (0.00035 mol) of 2,2'-azobismethylisobutyrate. Then, while the contents of the flask were being homogeneously stirred, nitrogen gas was blown into the solution to remove oxygen in the flask. Then, while stirring and blowing of nitrogen gas were being continued, the flask was placed in an oil bath set at 60 to 65°C to heat the contents, and kept at the temperature for 4 hours. Then, the temperature of the oil bath was set to 90°C to remove the normal hexane. Then, the contents of the flask were transferred into a vacuum dryer (Vacuum Drying Oven DP33 from Yamato Scientific Co., Ltd.), the temperature of the oven was set to 110°C and the pressure of the oven was set to 10 mmHg, and the contents were dried for 8 hours to obtain 45 g of an alkyl-modified carboxyl group-containing polymer (polymer 1) as a white powder.

### (Production Example 2)

Following the procedure of Production Example 1, except for changing the amount of BLEMMER VMA70 (NOF Corporation) used to 1.125 g, 45 g of an alkyl-modified carboxyl group-containing polymer (polymer 2) was obtained as a white powder.

### (Production Example 3)

Following the procedure of Production Example 1, except for changing the amount of BLEMMER VMA70 (NOF Corporation) used to 2.25 g, 46 g of an alkyl-modified carboxyl group-containing polymer (polymer 3) was obtained as a white powder.

### (Production Example 4)

Following the procedure of Production Example 1, except for changing the amount of BLEMMER VMA70 (NOF Corporation) used to 0.68 g, 43 g of an alkyl-modified carboxyl group-containing polymer (polymer 4) was obtained as a white powder.

### (Production Example 5)

Following the procedure of Production Example 1, except for changing the amount of BLEMMER VMA70 (NOF Corporation) used to 1.125 g, and changing the amount of the pentaerythritol allyl ether (a mixture of pentaerythritol triallyl ether and pentaerythritol tetraallyl ether) used to 0.135 g from 0.02 g, 45 g of an alkyl-modified carboxyl group-containing polymer (polymer 5) was obtained as a white powder.

### [Evaluation of Alkyl-Modified Carboxyl Group-Containing Polymers]

To evaluate the characteristics of the polymers 1 to 5 obtained in Production Examples 1 to 5 as thickeners, viscosities and light transmittances were measured for 1% by mass neutral viscous solutions of these polymers, as well as electrolyte-containing solutions prepared by adding sodium chloride to these 1% by mass neutral viscous solutions, followed by stirring.

### (1) Preparation of Evaluation Samples

Three grams of each of the polymers as evaluation samples was weighed out, and each polymer was gradually added into 285.9 g of deionized water and dispersed with stirring. Then, 11.1 g of a 6% by mass aqueous solution of sodium hydroxide was added while further stirring, and the mixture was stirred until the solution became homogeneous to prepare a 1% by mass neutral viscous solution of the polymer. These neutral viscous solutions had pHs of 6.0 to 6.5. Furthermore, 0.75 to 9 g of sodium chloride was added to 300 g of each of these neutral viscous solutions, stirred and dissolved to prepare an electrolyte-containing solution having a sodium chloride concentration of 0.25, 0.5, 0.75, 1, 2, or 3% by mass. In the following evaluation, the 1% by mass neutral viscous solutions and the electrolyte-containing solutions having the respective sodium chloride concentrations were allowed to stand for 1 hour after preparation, and then used as evaluation samples.

### (2) Viscosity Measurement

Using a BH-type rotational viscometer, the rotational speed of a spindle rotor No. 6 was set to 20 rotations per minute at 25°C, and the viscosity after 1 minute was measured for each evaluation sample. Table 1 shows the measurement results for the polymer 1, Table 2 for the polymer 2, Table 3 for the polymer 3, Table 4 for the polymer 4, and Table 5 for the polymer 5.

### (3) Light Transmittance Measurement

Each of the evaluation samples was defoamed by being subjected to an operation at 2000 rotations per minute for 5 minutes in a centrifuge, and then light transmittance was measured at a measurement wavelength of 425 nm, using a cell with a light path length of 1 cm. Typically, an evaluation sample having a light transmittance of 90% or more can be determined as being visually transparent. The measurement results are shown in Tables 1 to 5.

**[Table 1]**

| NaCl Concentration [%] | Viscosity [mPa·s] | Light Transmittance [%] |
|---|---|---|
| 0 | 270 | 98 |
| 0.25 | 10,350 | 98 |
| 0.5 | 15,900 | 98 |
| 0.75 | 15,000 | 98 |
| 1 | 14,500 | 97 |
| 2 | 13,350 | 94 |
| 3 | 3,950 | 60 |

**[Table 2]**

| NaCl Concentration [%] | Viscosity [mPa·s] | Light Transmittance [%] |
|---|---|---|
| 0 | 470 | 98 |
| 0.25 | 10,300 | 98 |
| 0.5 | 16,000 | 97 |
| 0.75 | 15,500 | 96 |
| 1 | 16,000 | 96 |
| 2 | 15,000 | 95 |
| 3 | 7,800 | 63 |

**[Table 3]**

| NaCl Concentration [%] | Viscosity [mPa·s] | Light Transmittance [%] |
|---|---|---|
| 0 | 900 | 99 |
| 0.25 | 25,800 | 98 |
| 0.5 | 20,700 | 96 |
| 0.75 | 17,500 | 95 |
| 1 | 14,000 | 93 |
| 2 | 3,500 | 70 |
| 3 | - | - |

**[Table 4]**

| NaCl Concentration [%] | Viscosity [mPa·s] | Light Transmittance [%] |
|---|---|---|
| 0 | 110 | 99 |
| 0.25 | 1,000 | 98 |
| 0.5 | 1,850 | 98 |
| 0.75 | 2,200 | 98 |
| 1 | 1,560 | 95 |
| 2 | 900 | 94 |
| 3 | 5,500 | 92 |

**[Table 5]**

| NaCl Concentration [%] | Viscosity [mPa·s] | Light Transmittance [%] |
|---|---|---|
| 0 | 32,300 | 97 |
| 0.25 | 24,250 | 77 |
| 0.5 | 18,300 | 60 |
| 0.75 | 12,160 | 53 |
| 1 | 8,380 | 43 |
| 2 | 1,900 | 21 |
| 3 | 500 | 2 |

### (Example 1)

Using an ultrahigh-speed stirring system T. K. ROBOMIX (Primix Corporation) equipped with a Disper blade (diameter: 40 mm), 10 g of the polymer 1 was gradually added into a 2-L beaker containing 977.7 g of distilled water and dispersed at a stirring rate of 5000 rotations per minute. After stirring was performed for 10 minutes at 5000 rotations per minute, the stirring rate was reduced to 3000 rotations per minute, and stirring was continued for 20 minutes. Then, at a stirring rate of 1400 rotations per minute, 12.3 g of an 18% by mass aqueous solution of sodium hydroxide was added to prepare a colorless and transparent neutral viscous solution. The neutral viscous solution had a pH of 6.4. Using a BH-type rotational viscometer, the rotational speed of a spindle rotor No. 5 was set to 20 rotations per minute at 25°C, and the viscosity after 1 minute was measured for this neutral viscous solution. As a result, the viscosity was 330 mPa·s (the content of an aqueous solution of a betaine-based emulsifier (having a solids content of about 30% by mass) was 0% by mass). This neutral viscous solution was divided into 144-g portions in 200-L beakers. Then, using four paddle blades (diameter: 50 mm) at a stirring rate of 500 rotations per minute, 6 g of a cocamidopropyl betaine solution (RIKABION B-200 from New Japan Chemical Co., Ltd., solids content: 31.5% by mass) as a betaine-based emulsifier was slowly added. The stirring rate was then increased to 1000 rotations per minute, and mixing was performed for 1 hour to prepare a gel composition (the content of the aqueous solution of the betaine-based emulsifier (having a solids content of about 30% by mass) was 4% by mass). Using a BH-type rotational viscometer, the rotational speed of a spindle rotor No. 7 was set to 20 rotations per minute at 25°C, and the viscosity after 1 minute was measured for the gel composition. As a result, the viscosity was 104,400 mPa·s. These results are shown in Table 6. Furthermore, gel compositions were prepared in the same manner such that the content of the aqueous solution of the betaine-based emulsifier (having a solids content of about 30% by mass) was 6 or 8% by mass, and the viscosities of these gel compositions were measured. The results are shown in Table 7.

### (Example 2)

Following the procedure of Example 1, except for changing the polymer 1 to the polymer 2, a gel composition was prepared, and the viscosities were measured. The results are shown in Tables 6 and 7.

### (Example 3)

Following the procedure of Example 1, except for changing the polymer 1 to the polymer 3, a gel composition was prepared, and the viscosities were measured. The results are shown in Tables 6 and 7.

### (Comparative Example 1)

Following the procedure of Example 1, except for changing the polymer 1 to the polymer 4, a gel composition was prepared, and the viscosities were measured. The results are shown in Tables 6 and 7.

### (Comparative Example 2)

Following the procedure of Example 1, except for changing the polymer 1 to the polymer 5, a gel composition was prepared, and the viscosities were measured. The results are shown in Tables 6 and 7.

**[Table 6]**

| | Gel Composition Viscosity [mPa·s] | |
|---|---|---|
| | Content of Cocamidopropyl Betaine Solution | |
| | 0% by mass | 4% by mass |
| Example 1 | 330 | 104,400 |
| Example 2 | 510 | 124,400 |
| Example 3 | 1,000 | 109,600 |
| Comparative Example 1 | 150 | 26,100 |
| Comparative Example 2 | 31,500 | 44,000 |

**[Table 7]**

| | Gel Composition Viscosity [mPa·s] | |
|---|---|---|
| | Content of Cocamidopropyl Betaine Solution | |
| | 6% by mass | 8% by mass |
| Example 1 | 73,000 | 41,050 |
| Example 2 | 94,000 | 60,500 |
| Example 3 | 67,000 | 34,500 |
| Comparative Example 1 | 25,050 | 14,000 |
| Comparative Example 2 | 24,800 | 4,300 |

### <Evaluation of Relationships between pH and Viscosity of Gel Compositions>

### (Example 4)

As in Example 1, using an ultrahigh-speed stirring system T. K. ROBOMIX (Primix Corporation) equipped with a Disper blade (diameter: 40 mm), 10 g of the polymer 1 was gradually added into a 2-L beaker containing 987.7 g of distilled water and dispersed at a stirring rate of 5000 rotations per minute. After stirring was performed for 10 minutes at 5000 rotations per minute, the stirring rate was reduced to 3000 rotations per minute, and stirring was continued for 20 minutes. Then, at a stirring rate of 1400 rotations per minute, 2.3 g of an 18% by mass aqueous solution of sodium hydroxide was added to prepare a neutral viscous solution having a pH of about 3.8. Next, the pH of the neutral viscous solution was changed as shown in Table 8, by adding predetermined amounts of an 18% by mass aqueous solution of sodium hydroxide, and homogeneously stirring the mixtures. Then, the viscosities at various pHs of the neutral viscous solution were measured (the content of cocamidopropyl betaine (solids content): 0% by mass (Referential) in Table 8). The results are shown in Table 8. The viscosities were measured in the same manner as in Example 1.

As in Example 1, a neutral viscous solution in which the polymer 1 was adjusted to a pH of about 3.8 was divided into 144-g portions in 200-L beakers. Then, using four paddle blades (diameter: 50 mm) at a stirring rate of 500 rotations per minute, cocamidopropyl betaine (RIKABION B-200 from New Japan Chemical Co., Ltd., solids content: 31.5% by mass) was slowly added such that each of the contents shown in Table 8 (the cocamidopropyl betaine content (solids content) was 0.25, 0.50, or 0.75% by mass for each case) was achieved. The stirring rate was then increased to 1000 rotations per minute, and mixing was performed for 1 hour to prepare each gel composition. Next, the pH of the gel composition was changed as shown in Table 8, by adding predetermined amounts of an 18% by mass aqueous solution of sodium hydroxide to the obtained gel composition, and homogeneously stirring the mixtures. Then, the viscosities at various pHs of the neutral viscous solution were measured. The results are shown in Table 8.

**[Table 8]**

| Example 4 (Content of Polymer 1 in Gel Composition is 1% by mass) | | | | | | | |
|---|---|---|---|---|---|---|---|
| Cocamidopropyl Betaine Content (Solids Content) 0% by mass (Referential) | | Cocamidopropyl Betaine Content (Solids Content) 0.25% by mass | | Cocamidopropyl Betaine Content (Solids Content) 0.50% by mass | | Cocamidopropyl Betaine Content (Solids Content) 0.75% by mass | |
| pH | Viscosity [mPa·s] | pH | Viscosity [mPa·s] | pH Viscosity [mPa·s] | | pH | Viscosity [mPa·s] |
| 3.81 | 100 | 3.95 | 15,000 | 3.98 | 4,100 | 4.33 | 3,140 |
| 4.01 | 172 | 4.50 | 39,200 | 4.39 | 31,200 | 4.57 | 22,400 |
| 4.23 | 372 | 4.95 | 47,000 | 4.95 | 53,800 | 5.10 | 42,000 |
| 4.42 | 910 | 5.25 | 54,600 | 5.24 | 63,200 | 5.62 | 51,200 |
| 4.62 | 1,850 | 5.69 | 63,000 | 5.43 | 64,400 | 5.93 | 74,200 |
| 4.84 | 3,500 | 5.98 | 72,000 | 5.73 | 76,600 | 6.50 | 102,000 |
| 5.00 | 4,600 | 6.20 | 76,000 | 5.96 | 99,200 | | |
| 5.20 | 4,920 | 6.52 | 68,000 | 6.23 | 112,000 | | |
| 5.31 | 5,020 | 6.78 | 52,000 | 6.45 | 94,200 | | |
| 5.64 | 1,540 | | | 6.76 | 77,000 | | |
| 5.77 | 960 | | | 6.96 | 58,400 | | |
| 5.95 | 450 | | | | | | |
| 6.26 | 272 | | | | | | |
| 6.39 | 250 | | | | | | |
| 6.58 | 220 | | | | | | |
| 6.75 | 180 | | | | | | |

As is evident from the results shown in Table 8, it is found that when the content of the betaine-based emulsifier (solids content) in a gel composition is set to about 0.2 to 0.8% by mass, a gel composition having a high viscosity even at a low pH in the range of about 4.4 to 5.5, for example, can be obtained. It is also found that because the difference in viscosity before and after addition of the betaine-based emulsifier is particularly large, a gel composition having a suitably high viscosity in the low pH range can be prepared by mixing the various components homogeneously at a low viscosity before addition of the betaine-based emulsifier, and then adding the betaine-based emulsifier. That is, it is found that, in the low pH range, before addition of the betaine-based emulsifier (the content of cocamidopropyl betaine (solids content): 0% by mass (Referential) in Table 8), the viscosity of each composition is very low, and the components can be readily homogeneously dispersed or dissolved; subsequently, a predetermined amount of the betaine-based emulsifier is added (for example, the content of cocamidopropyl betaine (solids content) in Table 8 is 0.25, 0.50, or 0.75% by mass), and thereby a gel composition having a high viscosity can be obtained.

### (Comparative Example 3)

Following the procedure of Example 4, except for using the polymer 5 instead of the polymer 1, a neutral viscous solution having a pH of about 3.8 was prepared. Next, the pH of the neutral viscous solution was changed as shown in Table 9, by adding predetermined amounts of an 18% by mass aqueous solution of sodium hydroxide, and homogeneously stirring the mixtures. Then, the viscosities at various pHs of the neutral viscous solution were measured (the content of cocamidopropyl betaine (solids content): 0% by mass (Referential) in Table 9). The results are shown in Table 9.

As in Example 4, a neutral viscous solution in which the polymer 5 was adjusted to a pH of about 3.8 was divided into 144-g portions in 200-L beakers. Then, using four paddle blades (diameter: 50 mm) at a stirring rate of 500 rotations per minute, cocamidopropyl betaine (RIKABION B-200 from New Japan Chemical Co., Ltd., solids content: 31.5%) was slowly added such that each of the contents shown in Table 9 (the cocamidopropyl betaine content (solids content) was 0.25, 0.50, or 0.75% by mass) was achieved. The stirring rate was then increased to 1000 rotations per minute, and mixing was performed for 1 hour to prepare each gel composition. Next, the pH of the gel composition was changed as shown in Table 9, by adding predetermined amounts of an 18% by mass aqueous solution of sodium hydroxide to the obtained gel composition, and homogeneously stirring the mixtures. Then, the viscosities at various pHs of the neutral viscous solution were measured. The results are shown in Table 9.

**[Table 9]**

| Comparative Example 3 (Content of Polymer 5 in Gel Composition is 1% by mass) | | | | | | | |
|---|---|---|---|---|---|---|---|
| Cocamidopropyl Betaine Content (Solids Content) 0% by mass (Referential) | | Cocamidopropyl Betaine Content (Solids Content) 0.25% by mass | | Cocamidopropyl Betaine Content (Solids Content) 0.50% by mass | | Cocamidopropyl Betaine Content (Solids Content) 0.75% by mass | |
| pH | Viscosity [mPa·s] | pH | Viscosity [mPa·s] | pH | Viscosity [mPa·s] | pH | Viscosity [mPa·s] |
| 3.99 | 25,800 | 4.10 | 21,300 | 4.37 | 14,100 | 3.95 | 3,500 |
| 4.56 | 27,800 | 4.25 | 25,500 | 5.10 | 45,200 | 4.34 | 12,000 |
| 5.15 | 27,800 | 4.52 | 35,000 | 5.62 | 66,000 | 4.43 | 13,200 |
| 5.70 | 26,900 | 4.84 | 46,000 | 6.07 | 85,800 | 4.70 | 19,550 |
| 6.45 | 25,900 | 5.09 | 52,600 | 6.53 | 98,000 | 4.97 | 27,000 |
| 6.82 | 25,600 | 5.57 | 60,000 | | | 5.32 | 35,700 |
| | | 5.94 | 73,000 | | | 5.66 | 60,000 |
| | | 6.43 | 77,000 | | | 5.89 | 86,000 |
| | | | | | | 6.17 | 109,000 |
| | | | | | | 6.40 | 122,000 |

As is evident from the results shown in Table 9, it is found that when the polymer 5 (a polymer having a high crosslinking degree, as compared to the polymer 1) is used, even though the content of the betaine-based emulsifier (solids content) in a gel composition is set to about 0.2 to 0.8% by mass, the viscosity of the composition tends to be lower than those in Example 4, at a low pH in the range of 4.4 to 5.5, for example. It is also found that because the viscosity before addition of the betaine-based emulsifier (the content of cocamidopropyl betaine (solids content): 0% by mass (Referential) in Table 9) is high in the low pH range, the components are less readily homogeneously dispersed or dissolved than in Example 4.

## Claims

1. A gel composition comprising:
0.1 to 2% by mass of a neutralized product of an alkyl-modified carboxyl group-containing polymer, which is a copolymer of monomers comprising 100 parts by mass of (meth)acrylic acid (a), 2.5 to 5 parts by mass of a (meth)acrylic acid alkyl ester (b) in which the alkyl group has 18 to 24 carbon atoms, and 0.1 part by mass or less of a compound (c) having two or more ethylenically unsaturated groups; and
a betaine-based emulsifier
wherein the content of the betaine-based emulsifier is 0.2 to 0.8% by mass, and the pH of the composition is 4.4 to 5.5.

2. The gel composition according to claim 1, wherein the alkyl-modified carboxyl group-containing polymer has a viscosity at 25°C of 1500 mPa·s or less and a light transmittance of 90% or more, when in the form of a 1% by mass neutral viscous solution, and
a solution prepared by adding 0.25 to 3 parts by mass of sodium chloride to 100 parts by mass of the 1% by mass neutral viscous solution has a maximum viscosity at 25°C of 15,000 to 40,000 mPa·s and a light transmittance of 90% or more.

3. The gel composition according to claims 1 or 2, wherein the compound (c) having two or more ethylenically unsaturated groups is at least one selected from the group consisting of pentaerythritol allyl ethers, diethylene glycol diallyl ether, polyethylene glycol diallyl ether, and polyallylsaccharose.

4. The gel composition according to any one of claims 1 to 3, wherein the betaine-based emulsifier is cocamidopropyl betaine.

5. A cosmetic preparation comprising the gel composition according to any one of claims 1 to 4.

6. A method for producing a gel composition comprising the steps of:
mixing an alkyl-modified carboxyl group-containing polymer, which is a copolymer of monomers comprising 100 parts by mass of (meth)acrylic acid (a), 2.5 to 5 parts by mass of a (meth)acrylic acid alkyl ester (b) in which the alkyl group has 18 to 24 carbon atoms, and 0.1 part by mass or less of a compound (c) having two or more ethylenically unsaturated groups, with an alkaline component, to prepare a neutralized product of the alkyl-modified carboxyl group-containing polymer; and
mixing the neutralized product of the alkyl-modified carboxyl group-containing polymer with a betaine-based emulsifier,
wherein the content of the neutralized product of the alkyl-modified carboxyl group-containing polymer in the gel composition is 0.1 to 2 % by mass,
the content of the betaine-based emulsifier in the gel composition is 0.2 to 0.8% by mass, and the pH of the composition is 4.4 to 5.5.

## Patentansprüche

1. Gelzusammensetzung, die Folgendes umfasst:
0,1 bis 2 Massen-% eines neutralisierten Produkts eines Alkyl-modifizierten Carboxylgruppen-hältigen Polymers, das ein Copolymer von Monomeren ist, die 100 Massenteile (Meth)acrylsäure (a), 2,5 bis 5 Massenteile (Meth)acrylsäurealkylester (b), worin die Alkylgruppe 18 bis 24 Kohlenstoffatome aufweist, und 0,1 Massenteile oder weniger einer Verbindung (c), die zwei oder mehr ethylenisch ungesättigte Gruppen aufweist, umfassen; und
einen Emulgator auf Betain-Basis;
wobei der Gehalt an Emulgator auf Betain-Basis 0,2 bis 0,8 Massen-% beträgt und der pH der Zusammensetzung 4,4 bis 5,5 beträgt.

2. Gelzusammensetzung nach Anspruch 1, wobei das Alkyl-modifizierte Carboxylgruppen-hältige Polymer bei 25 °C eine Viskosität von 1.500 mPa·s oder weniger und eine Lichtdurchlässigkeit von 90 % oder mehr aufweist, wenn es in Form einer 1-massen-%igen, neutralen, viskosen Lösung vorliegt, und
eine Lösung, die durch Zusetzen von 0,25 bis 3 Massenteilen Natriumchlorid zu 100 Massenteilen der 1-massen-%igen, neutralen, viskosen Lösung hergestellt wird, bei 25 °C eine maximale Viskosität von 15.000 bis 40.000 mPa·s und eine Lichtdurchlässigkeit von 90 % oder mehr aufweist.

3. Gelzusammensetzung nach Anspruch 1 oder 2, wobei die Verbindung (c), die zwei oder mehr ethylenisch ungesättigte Gruppen aufweist, zumindest eine, ausgewählt aus der aus Pentaerythritallylethern, Diethylenglykoldiallylether, Polyethylenglykoldiallylether und Polyallylsaccharose bestehenden Gruppe ist.

4. Gelzusammensetzung nach einem der Ansprüche 1 bis 3, wobei der Emulgator auf Betain-Basis Cocamidopropylbetain ist.

5. Kosmetikpräparat, das eine Gelzusammensetzung nach einem der Ansprüche 1 bis 4 umfasst.

6. Verfahren zur Herstellung einer Gelzusammensetzung, das die folgenden Schritte umfasst:
das Vermischen eines Alkyl-modifizierten Carboxylgruppen-hältigen Polymers, das ein Copolymer von Monomeren ist, die 100 Massenteile (Meth)acrylsäure (a), 2,5 bis 5 Massenteile (Meth)acrylsäurealkylester (b), in dem die Alkylgruppe 18 bis 24 Kohlenstoffatome aufweist, und 0,1 Massenteile oder weniger einer Verbindung (c), die zwei oder mehr ethylenisch ungesättigte Gruppen aufweist, umfassen, mit einer alkalischen Komponente zur Herstellung eines neutralisierten Produkts des Alkyl-modifizierten Carboxylgruppen-hältigen Polymers; und
das Vermischen des neutralisierten Produkts des Alkyl-modifizierten Carboxylgruppen-hältigen Polymers mit einem Emulgator auf Betain-Basis;
wobei der Gehalt an dem neutralisierten Produkt des Alkyl-modifizierten Carboxylgruppen-hältigen Polymers in der Gelzusammensetzung 0,1 bis 2 Massen-% beträgt, der Gehalt an dem Emulgator auf Betain-Basis in der Gelzusammensetzung 0,2 bis 0,8 Massen-% beträgt und der pH der Zusammensetzung 4,4 bis 5,5 beträgt.

## Revendications

1. Composition de gel comprenant :
0,1 à 2 % en masse d'un produit neutralisé d'un polymère contenant un groupe carboxyle modifié par un alkyle, qui est un copolymère de monomères comprenant 100 parties en masse d'acide (méth)acrylique (a), 2,5 à 5 parties en masse d'un ester alkylique d'acide (méth)acrylique (b) dans lequel le groupe alkyle a 18 à 24 atomes de carbone, et 0,1 partie en masse ou moins d'un composé (c) ayant deux groupes éthyléniquement insaturés ou plus ; et
un émulsifiant à base de bétaïne
dans laquelle la teneur en émulsifiant à base de bétaïne est de 0,2 à 0,8 % en masse, et le pH de la composition est de 4,4 à 5,5.

2. Composition de gel selon la revendication 1, dans laquelle le polymère contenant un groupe carboxyle modifié par un alkyle a une viscosité à 25°C de 1500 mPa.s ou moins et un facteur de transmission de lumière de 90 % ou plus, lorsque sous la forme d'une solution visqueuse neutre à 1 % en masse, et
une solution préparée en ajoutant 0,25 à 3 parties en masse de chlorure de sodium à 100 parties en masse de la solution visqueuse neutre à 1 % en masse a une viscosité maximum à 25°C de 15 000 à 40 000 mPa.s et un facteur de transmission de lumière de 90 % ou plus.

3. Composition de gel selon la revendication 1 ou 2, dans laquelle le composé (c) ayant deux groupes éthyléniquement insaturés ou plus est au moins un choisi dans le groupe constitué par de éthers allyliques de pentaérythritol, de l'éther diallylique de diéthylèneglycol, de l'éther diallylique de polyéthylèneglycol, et du polyallylsaccharose.

4. Composition de gel selon l'une quelconque des revendications 1 à 3, dans laquelle l'émulsifiant à base de bétaïne est la cocamidopropyl bétaïne.

5. Préparation cosmétique comprenant la composition de gel selon l'une quelconque des revendications 1 à 4.

6. Procédé de production d'une composition de gel comprenant les étapes consistant à :
mélanger un polymère contenant un groupe carboxyle modifié par un alkyle, qui est un copolymère de monomères comprenant 100 parties en masse d'acide (méth)acrylique (a), 2,5 à 5 parties en masse d'un ester alkylique d'acide (méth)acrylique (b) dans lequel le groupe alkyle a 18 à 24 atomes de carbone, et 0,1 partie en masse ou moins d'un composé (c) ayant deux groupes éthyléniquement insaturés ou plus, avec un composant alcalin, pour préparer un produit neutralisé du polymère contenant un groupe carboxyle modifié par un alkyle ; et
mélanger le produit neutralisé du polymère contenant un groupe carboxyle modifié par un alkyle avec un émulsifiant à base de bétaïne,
dans lequel la teneur en produit neutralisé du polymère contenant un groupe carboxyle modifié par un alkyle dans la composition de gel est de 0,1 à 2 % en masse,
la teneur en émulsifiant à base de bétaïne dans la composition de gel est de 0,2 à 0,8 % en masse, et le pH de la composition est de 4,4 à 5,5.
